# EUROPEAN PATENT SPECIFICATION

(11) **EP 3 813 936 B1**
(45) Date of publication and mention of the grant of the patent: **05.11.2025**
(21) Application number: 19814206.9
(22) Date of filing: 06.06.2019
(51) Int. Cl.: A61N 1/40, A61B 18/00, A61B 18/12, A61B 18/14, A61B 17/00

(54) **PORTABLE WOUND TREATMENT SYSTEM**
TRAGBARES WUNDBEHANDLUNGSSYSTEM
SYSTÈME PORTABLE DE TRAITEMENT DE PLAIE

(30) Priority: 06.06.2018 US 201862681352 P
(43) Date of publication of application: 05.05.2021
(73) Proprietor: Rioux, Robert F., Ashland, MA 01721 (US)
(72) Inventor: Rioux, Robert F., Ashland, MA 01721 (US)
(74) Representative: Barker Brettell LLP
(86) International application number: PCT/US2019/035780
(87) International publication number: WO 2019/236838

(56) References cited:
- WO-A1-2018/075389
- US-A- 6 142 992
- US-A1- 2003 216 729
- US-A1- 2016 317 221
- US-A1- 2018 014 880
- US-B2- 8 216 215

## Description

### Technical Field

The invention relates to a portable wound treatment system.

### Background

The treatment of wounds, both acute and chronic, involves various practices to remove barriers that impair wound healing. For example, a treatment may include wound bed preparation, debridement, control of the bacteria load in the wound, and management of exudate from the wound. Debridement generally describes the removal of devitalized tissue and removal of bacterial biofilm that increases bacterial resistance to antibiotics as well as the patient's immune response. The purpose of debridement is to restore the wound environment to that of an acute wound that is able to naturally progress toward complete healing. Although many approaches to debridement exist, surgical debridement remains the most common method. Surgical debridement generally involves the use of steel tools such as a curette, scissors, or scalpel that are used to clean or scrape the wound along with water for cleaning the wound. While current methods of surgical debridement remove visible necrotic tissues, such treatment methods may be painful and require anesthesia, and which may result in additional trauma to the wound environment and subsequent injury and/or infection, as well as incomplete healing.

Hydro-debridement is another debridement technique that involves use of a high pressure fluid stream running parallel to the wound surface to draw devitalized soft tissues into a cutting chamber for excision and removal. While seen as an improvement to surgical debridement, hydro-debridement has been found to contribute to bacteria aerosol spread due to the horizontal movement of fluid particles across the surface of the wound. Thus, current debridement techniques still result in inadequate biofilm control, recurrent infections that necessitate repetitive antibiotic treatments, hindered re-epithelialization, and ultimately, delayed healing and wound closure. US2018/014880 A1 discloses an electrosurgical system including an electrosurgical device to be delivered to a wound site to provide chronic wound treatment. The device can be used during a wound care procedure to provide targeted energy at a wound site for reducing the accumulation of biofilm present and removing necrotic tissue and debris so as to promote, stimulate, and stabilize the wound healing process. The device can further be used during a surgical procedure, such as preparation for an orthopedic implant, in which the device is configured to selectively coagulate one or more pockets prepared within bone tissue for holding an implant so as to prevent or stop fluid accumulation (e.g., blood from vessel(s)) as a result of the implant preparation. US2016/317221 A1 discloses a tissue ablation system including an ablation device having a deployable applicator head configured to be delivered to a tissue cavity and ablate marginal tissue surrounding the tissue cavity. The deployable applicator head is configured to be delivered to a tissue cavity while in a collapsed configuration and ablate marginal tissue surrounding the tissue cavity while in an expanded configuration. WO2018/075389 A1 discloses a medical device for providing treatment to diseased tissue and cells. The medical device is configured to ablate a target tissue surface, optionally within a resection cavity, and further deliver a therapeutic that targets diseased (e.g., cancer) cells via a marker whose expression is upregulated by the ablation. The ablation directly kills diseased cells associated with the tissue surface. While some diseased cells evade direct ablation, those cells nevertheless upregulate certain cell surface markers in response to the ablation, even while other, healthy or normal cells do not upregulate expression of the marker in response to the ablation. US8216215 B2 discloses a skin treatment apparatus that includes, a disposable electrode carrier with a plurality of voltage-applying dome-shaped elements protruding from the surface of the electrode carrier. Further, the protruding elements are spaced apart in a pattern. The apparatus operates to apply a voltage to at least some of the protruding elements. The apparatus applies a voltage to the protruding elements with a magnitude that is sufficient to result in an electrical break down of the skin and thereby cause electric current enabling the desired treatment. US6142992 A discloses a high frequency power supply for applying electrical energy to a target site on or within a patient's body includes an electrical output driver, an output current sensor detecting the current output from the driver, and a power limiting device coupled to the current sensor during normal conditions, the power limiting device operates on a continuous basis. When current output exceeds a predetermined threshold level, the power limiting device is adapted to reduce power on the output driver to a standby mode. The power limiting device operates on a periodic detection or duty cycle when in the standby mode. The power limiting device switches into the stand-by mode to prevent excessive power drains. The power supply operates at a low power, pulsatile manner when an attached probe is in conductive or isotonic fluid but is not engaging body tissue or near a high impedance source. In this pulsatile mode, the power supply operates in a cyclical manner, typically at a predetermined duty cycle. US2003/216729 A1 discloses a device for treating tissue in an individual to effect a weld between said tissue and at least one substrate comprising a material which functions as a fusion composition between the tissue and the substrate(s); a conducting element; a means of delivering a high frequency voltage or current or radiofrequency energy to the conducting element; and a means to control the extent of weld effected.

### Summary

The invention is defined in independent claim 1, and the dependent claims set out optional features. Wound healing involves a complex and dynamic process of angiogenesis, cell proliferation, deposition of an extracellular matrix, and wound contraction. In this process devitalized tissue acts as a physical barrier to re-epithelialization and prevents applied topical therapeutics from directly contacting the target tissue to provide its beneficial properties. As an example of devitalized tissue, the presence of necrotic tissue in the wound environment prevents angiogenesis, granulation tissue formation, epidermal regeneration, and normal extracellular matrix (ECM) formation. Further, devitalized tissue can serve as a nutrient source for bacteria, which assists in biofilm formation and resistance to antibiotics. Thus, debridement is an important step in wound treatment and preparation of the target tissue for re-epithelialization.

The present invention involves a portable wound treatment system that includes a disposable treatment device and a portable generator. The portable wound treatment system provides coagulation and/or ablation of a target tissue to thereby promote wound healing and facilitate debridement of the target tissue to remove devitalized tissue, remove bacterial biofilm, and control the bacterial load. As such, a portable wound treatment system may according to the invention help to accelerate wound closure by promoting dermal cell proliferation, collagen synthesis, and epidermal regeneration.

The treatment device includes a handle, an interior cavity for receiving a conductive fluid (e.g., a saline solution), a conductive element disposed within the interior cavity, and a plurality of apertures configured to allow passage of the conductive fluid from the interior cavity. The portable generator of the portable wound treatment system is configured to generate and transmit energy to the conductive element such that the conductive element conducts energy to be carried by the conductive fluid passing through one or more of the plurality of apertures to treat a target tissue contacted by that conductive fluid.

In one example, the portable generator may generate radiofrequency (RF) energy and the conductive element may be a bipolar helical coil. In this example, the portable wound treatment system may be employed to provide non-heat driven soft tissue dissolution using bipolar RF energy through a conductive fluid (e.g., a saline solution), although other suitable biocompatible conductive fluids that can effectively carry energy from the conductive element to the target tissue may be used. When a current from the portable generator is transmitted to the bipolar helical coil in the interior cavity of the treatment device, the current breaks the saline into sodium and chloride ions, which then form a plasma field about the plurality of apertures. The plasma field produced by these highly energized ions is sufficiently strong to break organic molecular bonds within soft tissue to thereby cause its dissolution. Accordingly, the portable wound treatment system may dissolve and remove devitalized or necrotic tissue, as well as bacterial biofilm, with limited thermal effect, resulting in minimal or no collateral damage to tissues adjacent to the target tissue.

Current plasma-mediated coagulation/ablation technologies typically employ RF generators that are large, heavy, and not portable. For example, current RF generators may be about 45 pounds or greater, or about 26 pounds or greater, and may be mounted to a wheeled cart due to their weight and bulk. Such RF generators may have dimensions of, for example, about 24 inches in height, 12 inches in length, and 14 inches in depth, or greater. As such, patients may only benefit from current plasma-mediated coagulation/ablation technologies by traveling to a hospital or clinic setting to receive the treatment. This limitation makes such treatments expensive and often restricts or prevents patient access to its wound-healing benefits.

In contrast, the present portable wound treatment system enables patients to receive plasma-mediated coagulation/ablation treatments in many settings, such as home visits by a nurse or even by the patients themselves with proper instruction. In various embodiments, the portable generator may have a weight of about 10 pounds or less, or about 5 pounds or less.

Also, in some embodiments, the portable generator may have dimensions of about 5 inches in height, 10 inches in length, and 8 inches in depth, or less, or dimensions of about 3 inches in height, 8 inches in length, and 6 inches in depth, or less.

The portable generator may include a thermoplastic housing as opposed to the metal housings currently used for generators. The portable generator also includes an improved transformer that weighs less than a traditional transformer. Also, the portable generator may include a shortened power cable that is about 3 feet long to carry the RF energy from the portable generator to the treatment device. This is an improvement over current RF generators that often have power cables of over 10 feet in length because approximately 10% of energy is lost for every foot of cable used, resulting in great energy inefficiency and making it necessary for the generator to be more powerful (and consume more energy) to compensate for the loss of transmitted energy due to the cable.

**In** various embodiments, each of the apertures are sized such that the conductive fluid does not flow from the plurality of apertures due to gravity alone. For example, each of the plurality of apertures may be sized such that the surface tension of the conductive fluid prevents droplets of the fluid from passively exiting the apertures. **In** one example, the portable wound treatment system further includes a reservoir operable to contain the conductive fluid and to convey the conductive fluid to the conductive element. **In** this example, the portable wound treatment system may include an actuator operable to control pressurized flow of the conductive fluid to the conductive element from the reservoir. For instance, the actuator may be a foot pedal, a control panel, a button, a motorized dispensing device, or any other device operable to control pressurized flow of the conductive fluid from the reservoir to the conductive element. **In** certain embodiments, the conductive fluid may only pass through one or more of the plurality of apertures when pressurized flow of the conductive fluid to the conductive element is provided.

The treatment device may be disposable such that the a nurse may select a treatment device among a variety of different sized and/or shaped treatment devices, each customized to treat a particular body part, type of tissue, or type of wound, for example. Because the portable wound healing system is designed to be used outside of an operating room or other treatment facility (and thus typically is not used in a sterile field), making the treatment device disposable helps prevent contamination and inadvertent spread of bacteria amongst patients or amongst wounds. Thus, the treatment device should be made as inexpensively as possible, such as from a biocompatible plastic or like materials, in order to make the treatment device lightweight and also disposable.

**In** certain embodiments, the treatment device may further include a plate coupled to a distal end of the handle and enclosing the interior cavity to thereby retain conductive fluid within the interior cavity, in which the plate has an exterior surface and comprises the plurality of apertures. **In** such embodiments, the exterior surface of the plate may contact and provide treatment to a target tissue adjacent to the exterior surface. Accordingly, in certain embodiments, the treatment device may be formed as a single disposable piece. In other embodiments, the treatment device may be formed as a handle and a separate plate that is then coupled to the handle.

In one example, the exterior surface of the plate further comprises protrusions that facilitate debridement of the target tissue as the exterior surface of the plate contacts the target tissue. The protrusions may of any shape such as contoured studs, lines, or staggered rows. As the exterior surface of the plate passes over a target tissue, the protrusions may mechanically assist in removal of biofilm and devitalized tissue that would hinder wound healing and closure.

In one example, the portable wound treatment system further comprises a non-conductive pad coupled to the exterior surface of the plate, in which the non-conductive pad includes an opening shaped or sized to correspond to a surface area of the target tissue or a dressing applied to the target tissue. The non-conductive pad may be provided without an existing opening such that a nurse or patient may cut an opening appropriately shaped or sized to correspond to a wound on a target tissue. Alternatively, the non-conductive pad may be provided with one or more existing openings and a non-conductive pad may be selected based on characteristics of the wound, the target tissue, or the treatment plan. By coupling the non-conductive pad to the exterior surface of the plate prior to treatment, the treatment device may selectively treat only a portion of a target tissue at a time (i.e., the portion of the target tissue corresponding to the position of the opening).

In some embodiments, the portable wound treatment system further includes a foam dressing that is applied to cover the target tissue prior to treatment with the wound treatment system, and to thereby improve conductivity to the target tissue, through the foam dressing. For example, a foam dressing shaped as an oval may be used to cover a generally oval shaped wound on a target tissue. In this example, a conductive medium, such as a 0.9% saline gel, may be applied to the foam dressing to improve conductivity between the exterior surface of the plate and the target tissue, through the foam dressing. The saline gel may also be selectively applied to only certain portions of the foam dressing to improve conductivity to only those portions.

The portable wound treatment system can include a suction device in fluid connection with the interior cavity via tubing coupled to the interior cavity. Any suitable tubing may be used to place the suction device in fluid connection with the interior cavity, such as rubber tubing, polyurethane tubing, PVC tubing, nylon tubing, polyethylene tubing, PTFE tubing, and the like. The portable wound treatment system may further include a controller operable to adjust negative pressure produced by the suction device. Any suitable controller that allows modulation of the negative pressure applied by the suction device to the treatment device may be used. For example, the controller may be a foot pedal, a knob, a control panel, a button, a joystick, or the like. In certain embodiments, the suction device is operable to remove debris from the target tissue, as the target tissue is treated by the wound treatment system, by applying negative pressure to the treatment device. For example, such debris may include devitalized tissue, necrotic tissue, biofilm, bacterial colonies, exudate from the wound, and the like. Similarly, the suction device may be used to remove excess conductive fluid from the target tissue.

### Brief Description of the Drawings

Features and advantages of the claimed subject matter will be apparent from the following detailed description of embodiments consistent therewith, which description should be considered with reference to the accompanying drawings.
FIG. 1 is a schematic illustration of a portable wound treatment system, consistent with the present disclosure;
FIG. 2 shows a perspective view of the portable wound treatment system;
FIG. 3 shows a side cross-section view of a treatment device of the portable wound treatment system;
FIG. 4 shows a perspective view of the treatment device;
FIG. 5A shows a side cross-section view of a treatment device of the portable wound treatment system;
FIG. 5B shows an enlarged portion of the plate of treatment device;
FIG. 6 shows a side cross-section view of a treatment device having a suction line;
FIGS. 7A-7C illustrate use of the portable wound treatment system with different types of wound dressings;
FIG. 8A shows a perspective view of a non-conductive pad;
FIG. 8B shows a schematic view of the non-conductive pad attached to the plate during treatment; and
FIG. 9 shows a perspective view of an exemplary portable generator consistent with the present disclosure.

For a thorough understanding of the present disclosure, reference should be made to the following detailed description, including the appended claims, in connection with the above-described drawings. Although the present disclosure is described in connection with exemplary embodiments, the disclosure is not intended to be limited to the specific forms set forth herein. It is understood that various omissions and substitutions of equivalents are contemplated as circumstances may suggest or render expedient.

### Detailed Description

The invention relates to a portable wound treatment system that includes a treatment device and a portable generator. The treatment device may be a disposable handheld device that includes a handle, an interior cavity for receiving a conductive fluid (e.g., a saline solution), a conductive element disposed within the interior cavity, and a plurality of apertures configured to allow passage of the conductive fluid from the interior cavity. The portable generator (e.g., a portable RF generator) of the portable wound treatment system is configured to generate and transmit energy to the conductive element such that the conductive element conducts energy to be carried by the conductive fluid passing through one or more of the plurality of apertures to treat a target tissue contacted by that conductive fluid. Accordingly, the portable wound treatment system may provide coagulation and/or ablation of a target tissue adjacent to the plurality of apertures.

The portable wound treatment system provides coagulation and/or ablation of a target tissue to thereby promote wound healing and provide debridement of the target tissue to remove devitalized tissue and bacterial biofilm, and to control the bacterial load. Accordingly, the portable wound treatment system may accelerate wound closure by promoting dermal cell proliferation, collagen synthesis, and epidermal regeneration. In addition, the portable wound treatment system may provide such coagulation and/or ablation treatment to a target tissue in various settings that do not require the patient to travel to a hospital or a clinic. For example, the portable generator may have a weight of about 5 pounds or less and dimensions of about 3 inches in height, 8 inches in length, and 6 inches in depth, or less. Thus, the treatment device, portable generator, and other components described herein may easily be transported and used in a home environment, improving patient access to the benefits of wound treatment by plasma-mediated coagulation and/or ablation.

A detailed description of the present invention is disclosed herein. It should be understood that the embodiments described are exemplary and should not be interpreted as limiting the scope of the invention. The detailed description disclosed herein is merely intended to teach one skilled in the art how to make and/or use the invention.

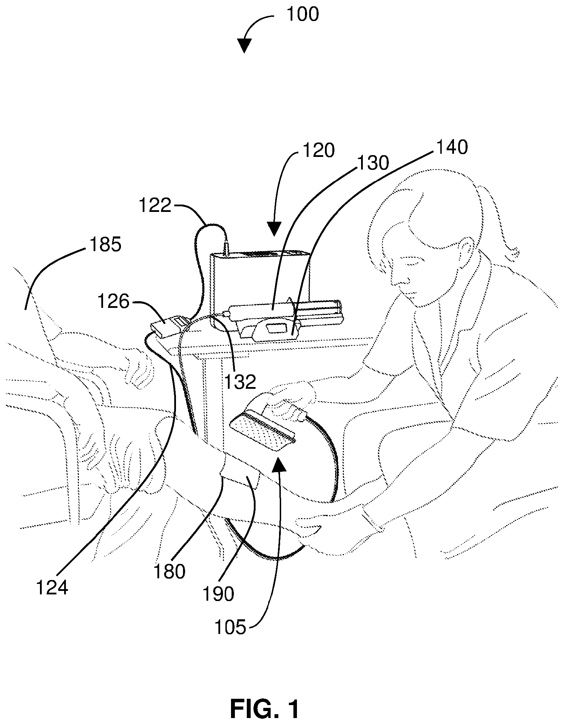

FIG. 1 is a schematic illustration of a portable wound treatment system 100 having a treatment device 105 connected to a reservoir 130 via a fluid line 132, and to a portable generator 120 via power cables 122 and 124. Although treatment device 105 is shown with a power cable 122 from portable generator 120 connected to power cable 124 from the treatment device at junction 126, the treatment device may alternatively be connected to portable generator 120 directly or by multiple power cables. In this embodiment, the portable wound treatment system 100 includes an actuator 140 connected to reservoir 130, in which the actuator is operable to control pressurized flow of a conductive fluid to the conductive element of the treatment device 105 from the reservoir 130. In this example, actuator 140 is a motorized device that may be used to depress a plunger on a syringe (i.e., reservoir 130) at a specified rate. Reservoir 130 is operable to contain a conductive fluid and to convey the conductive fluid to a conductive element of treatment device 105. For example, the conductive fluid may be any volume of a saline solution, such as 50 mL of a 0.9% saline solution. The actuator may be a foot pedal, a control panel, a button, a motorized dispensing device, or any other device operable to control pressurized flow of the conductive fluid from the reservoir to the conductive element.

The portable generator 120 is operable to generate and transmit energy to a conductive element of treatment device 105 such that the conductive element conducts energy to be carried by the conductive fluid contained within an interior cavity of the treatment device and passing through one or more of the plurality of apertures, as described below, for coagulation and/or ablation of a target tissue adjacent to the plurality of apertures.

In this illustration, the portable wound treatment system 100 is positioned on a table or flat surface and may be used in an at-home setting to treat target tissue 180 of patient 185, even though standard dressing 190 (e.g., a foam bandage). Although treatment device 105 is shown being used by nurse or medical practitioner, the patient 185 may also perform the treatment themselves, with proper instruction, and without traveling to a hospital or clinic.

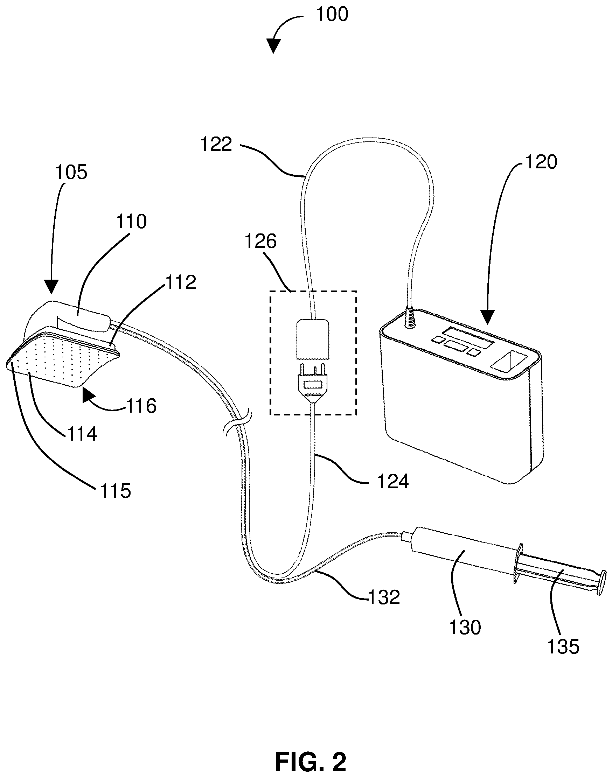

FIG. 2 shows a perspective view of the portable wound treatment system 100, which includes a treatment device 105 having a handle 110 and an interior cavity 112 for receiving a conductive fluid (e.g., a saline solution) within. In this embodiment, the treatment device 105 further includes a plate 114 coupled to the distal end of the treatment device, opposite the handle 110, and enclosing the interior cavity 112 to thereby retain the conductive fluid within the interior cavity. The plate 114 includes a plurality of apertures 116 configured to allow passage of the conductive fluid from the interior cavity 112 to an exterior surface 115 of the plate 116. In this embodiment, the exterior surface 115 contacts and provides treatment to a target tissue adjacent to the exterior surface. In this example, the exterior surface 115 of the plate 114 is contoured to facilitate engagement with the target tissue.

As shown, the treatment device 105 is connected to a portable generator 120 by power cables 122 and 124, which are connected at junction 126. In certain embodiments, the portable generator 120 may generate and transmit radiofrequency (RF) energy to a conductive element housed within the interior cavity 112 of the treatment device 105. The conductive element may then conduct the RF energy to conductive fluid that is initially contained within reservoir 130, and conveyed to the conductive element of the treatment device 105 via fluid line 132.

Also, as shown, the reservoir 130 resembles a syringe containing a saline solution, and the syringe is coupled to an actuator 135. In this example, the actuator 135 is a plunger that may be depressed to control pressurized flow of the conductive fluid to the conductive element from the reservoir 130. When pressurized flow of conductive fluid is provided to the treatment device 105, the conductive fluid may pass through one or more of the plurality of apertures 116 of the plate 114.

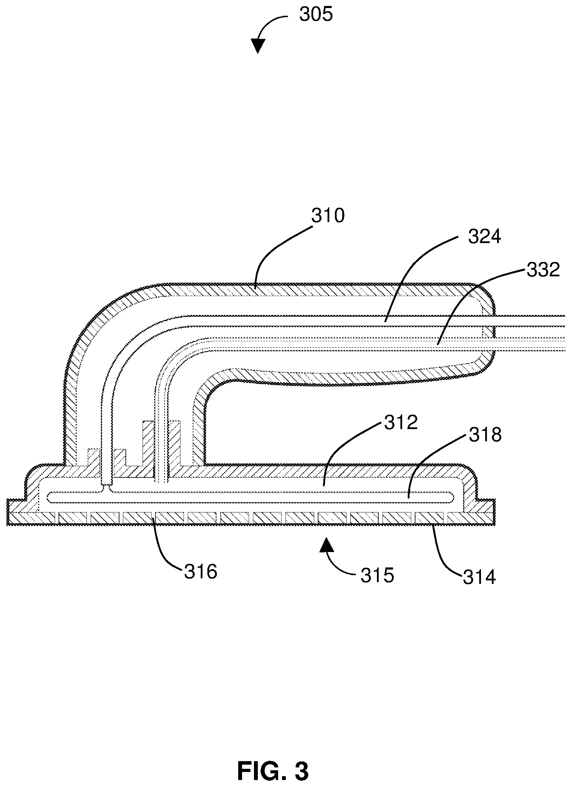

FIG. 3 shows a side cross-section view of a treatment device 305 having a handle 310 and an interior cavity 312 for receiving a conductive fluid within. The treatment device 305 further includes a conductive element 318 positioned within the interior cavity 312, and a plate 314 coupled to the distal end of the treatment device, opposite the handle, and enclosing the interior cavity 312 to thereby retain conductive fluid within the interior cavity. In this example, the plate 314 includes a plurality of apertures 316 configured to allow passage of the conductive fluid from the interior cavity 312 to the exterior surface 315 of the plate. In this example, treatment device 305 is connected to a reservoir (not shown) via fluid line 332 such that conductive fluid may be conveyed to interior cavity 312, wherein conductive element 318 is contained.

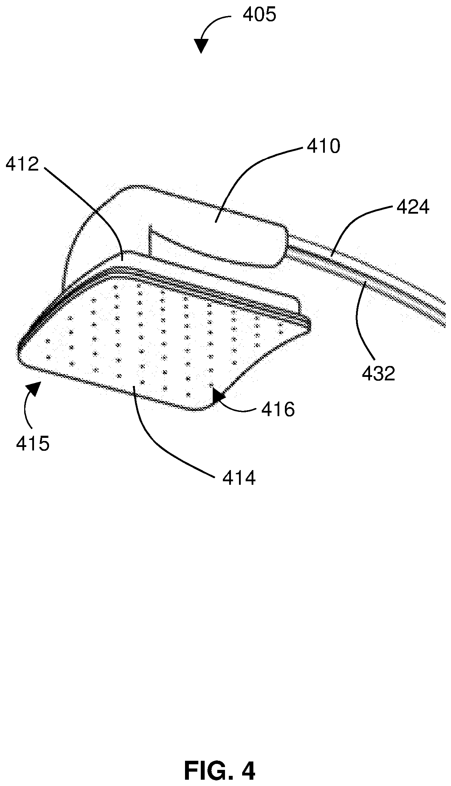

FIG. 4 shows a perspective view of the treatment device 405 having a handle 410 and an interior cavity 412 for receiving a conductive fluid. In this example, the treatment device 405 further includes plate 414 coupled to the distal end of the treatment device, opposite the handle, and enclosing the interior cavity 412 to thereby retain conductive fluid within the interior cavity 412. As shown, the plate 414 includes an exterior surface 415, through which a plurality of apertures 416 extends. The plurality of apertures 416 is configured to allow passage of the conductive fluid from the interior cavity 412 to the exterior surface 415 of the plate 414.

In this example, treatment device 405 is connected to a reservoir (not shown) via fluid line 432 such that conductive fluid may be conveyed to interior cavity 412, wherein a conductive element is contained. Treatment device 405 is also connected to a portable generator (not shown) via a power cable 424 such that energy may be transmitted from the portable generator to a conductive element of treatment device 405, and carried by the conductive fluid passing through one or more of the plurality of apertures 416 of plate 414 for coagulation and/or ablation of a target tissue adjacent to the exterior surface 415 of the plate.

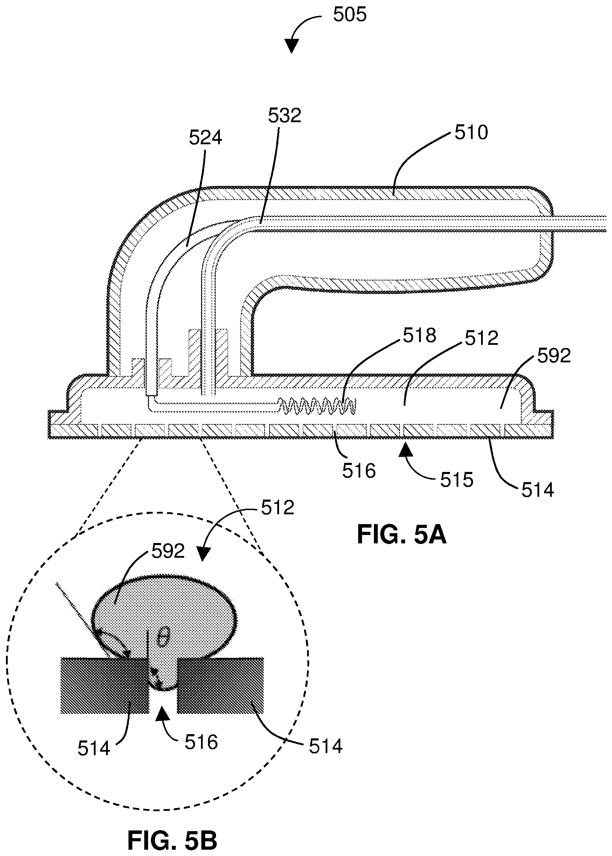

FIG. 5A shows a side cross-section view of a treatment device 505 having a handle 510 and an interior cavity 512 for receiving a conductive fluid. In this example, the treatment device 505 further includes plate 514 coupled to the distal end of the treatment device and enclosing the interior cavity 512 to thereby retain conductive fluid within the interior cavity 512. The plate 514 includes an exterior surface 515, through which a plurality of apertures 516 extends. The plurality of apertures 516 is configured to allow passage of the conductive fluid from the interior cavity 512 to the exterior surface 515 of the plate 514.

As shown, the treatment device 505 includes a bipolar helical coil as a conductive element 518 contained within the interior cavity 512, within which conductive fluid 592 may also be held. The conductive fluid may be conveyed to the interior cavity 512 from a reservoir via fluid line 532, as described above. The treatment device 505 may receive energy, such as RF energy from a portable generator and convey that energy to conductive element 518 and to the conductive fluid 592 contained within interior cavity 512. The RF energy may be carried by the conductive fluid passing through one or more of the plurality of apertures 516 for coagulation and/or ablation of a target tissue adjacent to the plurality of apertures 516 (i.e., the exterior surface 515 of the plate 514).

FIG. 5B shows an enlarged portion of the plate 514 of treatment device 505. As shown, the plurality of apertures 516 of the plate 514 are sized such that the droplet or portion of conductive fluid 592 does not flow from the plurality of apertures 592 due to gravity alone. For example, each of the plurality of apertures 516 may be sized such that the surface tension of the conductive fluid 592 prevents droplets of the fluid from passively exiting the apertures of the plate 514. However, as described above, the conductive fluid may pass through one or more of the plurality of apertures 516 of the plate 514 when pressurized flow of the conductive fluid 592 to the conductive element is provided.

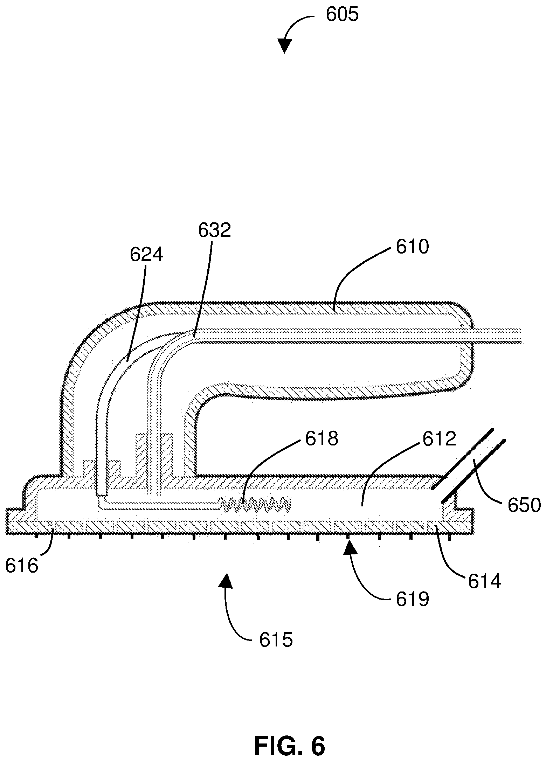

FIG. 6 shows a side cross-section view of a treatment device 605 connected to a suction line 650. In this example, the portable wound treatment system further includes a suction device (not shown) in fluid connection with the interior cavity 612 of treatment device 605 via suction line 650. As shown, the suction line 650 is directly connected to the interior cavity 612, but in other embodiments, suction line 650 may be indirectly connected to interior cavity 612. Any suitable tubing may be used as a suction line 650 to place the suction device in fluid connection with the interior cavity 612, such as rubber tubing, polyurethane tubing, PVC tubing, nylon tubing, polyethylene tubing, PTFE tubing, and the like. Any suitable suction device may be used, provided that the suction device is operable to apply negative pressure to the treatment device 605. For example, the suction device may be a piston, a bellows pump, or other vacuum device.

In this embodiment, treatment device 605 includes a plate 614 coupled to the distal end of the handle, in which the plate 614 further includes one or more protrusions 619 that facilitate debridement of a target tissue as the exterior surface 615 of the plate 614 contacts the target tissue. The protrusions 619 may of any shape such as contoured studs, lines, or staggered rows. As the exterior surface 615 of the plate 614 passes over a target tissue, the protrusions 619 may mechanically assist in removal of biofilm and devitalized tissue that would hinder wound healing and closure. In certain embodiments, the suction device is operable to remove debris from the target tissue, as the target tissue is treated by the wound treatment system, by applying negative pressure to the treatment device, such as the exterior surface 615 of the plate 614. For example, such debris may include devitalized tissue, necrotic tissue, biofilm, bacterial colonies, exudate from the wound, and the like.

The portable wound treatment system may further include a controller operable to adjust negative pressure produced by the suction device. Any suitable controller that allows modulation of the negative pressure applied by the suction device to the treatment device may be used. For example, the controller may be a foot pedal, a knob, a control panel, a button, a joystick, or the like. Similarly, the suction device may be used to remove excess conductive fluid from the target tissue.

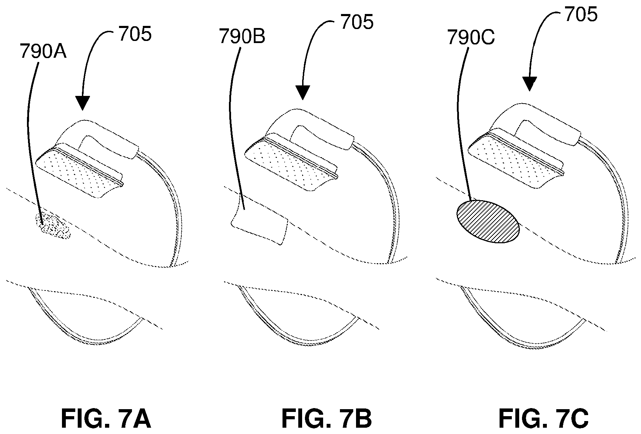

FIGS. 7A-7C illustrate use of the portable wound treatment system with different types of wound dressings.

FIG. 7A illustrates treatment device 705 being applied to a wound dressing 790A shaped or sized to correspond to a target tissue covered by the wound dressing.

FIG. 7B illustrates treatment device 705 being applied to a foam dressing 790B. In some embodiments, the portable wound treatment system further includes a foam dressing 790B that is applied to cover the target tissue prior to treatment with the wound treatment system, to thereby improve conductivity to the target tissue, through the foam dressing 790B. For example, a foam dressing 790B may be used to cover a wound on a target tissue, and a conductive medium, such as a 0.9% saline gel, may be applied to the foam dressing 790B to improve conductivity between the exterior surface of the plate and the target tissue, through the foam dressing. The saline gel may also be selectively applied to only certain portions of the foam dressing to improve conductivity to only those portions.

As described above, the treatment device provides coagulation and/or ablation of a target tissue to thereby promote wound healing by forming a plasma field about the plurality of apertures of the plate. The energy in the plasma field formed is sufficiently strong to break organic molecular bonds within soft tissue to thereby cause its dissolution. Accordingly, the portable wound treatment system is able to coagulate and/or ablate through existing dressings due to this virtual electrode design, in which energy is conducted to the target tissue without direct contact between the conductive element and the target tissue.

FIG. 7C illustrates treatment device 705 being applied to an optimized dressing 790C. Optimized dressing 790C may be modified to have different conductivity, insulation, or other characteristics, as compared to a dressing lacking such modifications. As shown, optimized dressing 790C shaped and sized to be used to cover a generally oval shaped wound on a target tissue. In this example, optimized dressing 790C has had a conductive medium, such as a 0.9% saline gel, applied to it to improve conductivity between the plurality of apertures of the treatment device and the target tissue, through the optimized dressing 790C. In one example, the optimized dressing may be a foam dressing having saline gel applied to one or both of its surfaces. The saline gel may also be selectively applied to only certain portions of the foam dressing to improve conductivity to only those portions. The conductive medium may also include one or more excipients to improve its biocompatibility or conductivity, or to enhance the moisture retention of the optimized dressing 790C, for example.

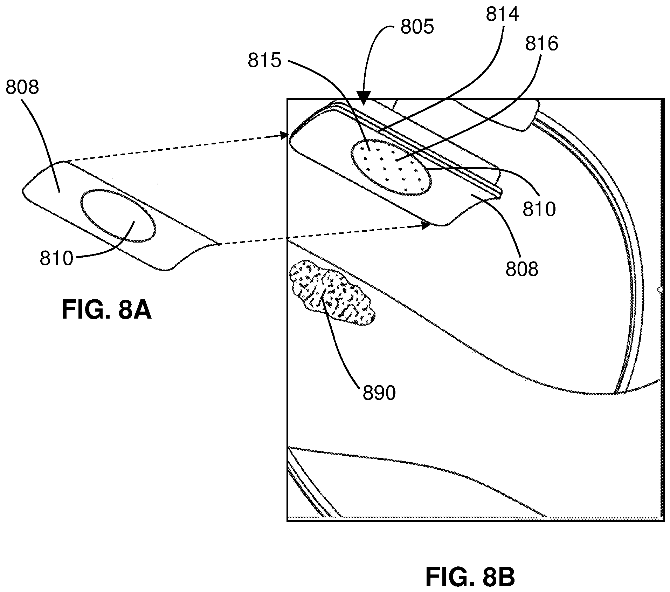

FIG. 8A shows a perspective view of a non-conductive pad 808. As shown, the non-conductive pad 808 includes an opening 810. The non-conductive pad 808 can be made of any suitable non-conductive material, such as a biocompatible rubber, Teflon, polyethylene, or the like. Also, the non-conductive pad 808 may be provided without an existing opening such that a nurse or patient may cut an opening 810 appropriately shaped or sized to correspond to a wound or a certain surface area on a target tissue. Alternatively, the non-conductive pad 808 may also be provided with an existing opening 810 and a non-conductive pad 808 may be selected based on specific considerations, such as characteristics of the wound, the target tissue, or the treatment approach. By coupling the non-conductive pad 808 to the plurality of apertures prior to treatment, the treatment device may selectively treat only a portion of a target tissue.

FIG. 8B shows a schematic view of the non-conductive pad 808 attached to the exterior surface 815 of a plate 814, as configured during treatment. Although non-conductive pad 808 is shown attached to the exterior surface of plate 814, in other embodiments, the non-conductive pad may be applied directly over the plurality of apertures of the treatment device 801. As shown, opening 810 is oval shaped to generally correspond to the shape of the wound dressing 890. In various embodiments, one or more openings of any shape or size may be provided in the non-conductive pad 808. For example, one or more openings that are circular, oval, rectangular, an irregular shape, or cut to correspond to a portion of a target tissue may be provided. As shown, a portion of the plurality of apertures 816 of plate 814 are revealed at opening 810 and the remainder of the apertures 816 are covered by the non-conductive pad 808, thereby allowing the treatment device to selectively treat a surface area of the target tissue corresponding to the area of opening 810.

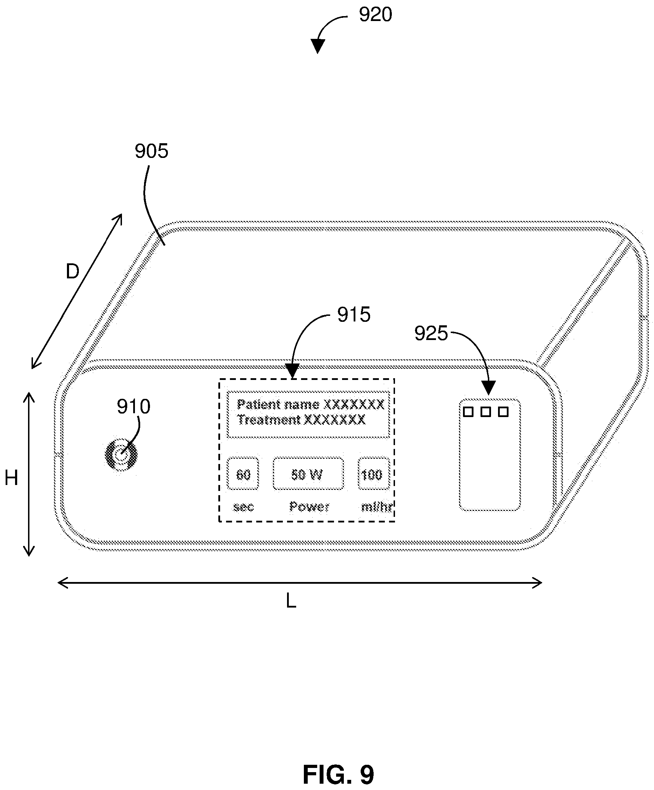

FIG. 9 shows a perspective view of an exemplary portable generator 920 having a housing 905, controls 925 for adjusting parameters of the portable generator 920 such as output wattage, a display 915, and a power cable connection 910. Display 915 may visually indicate to a user, information such as patient information, treatment-specific parameters, time elapsed during a treatment, and metrics of the energy transmitted. In various embodiments, portable generator 920 may also include a warning device to indicate to the user that a certain amount of time during treatment has elapsed, an output of energy has been reached during treatment, or like information. The warning device may provide such indications to the user in any suitable manner, such as an auditory or visual cue.

As described above, current plasma-mediated coagulation/ablation technologies typically employ RF generators that are large, heavy, and not portable. As a result, many patients lack access to this expensive and limited, but beneficial, technology that is generally restricted to use in a hospital or clinic setting. However, the portable generator 920 is an RF generator that is relatively small and lightweight, and that minimizes energy inefficiency compared to current RF generators. The portable generator 920 may easily be carried to a home environment for a treatment at the same time as other components of the portable wound treatment system described herein.

For example, the portable generator 920 may include a thermoplastic housing as opposed to the metal housings currently used for generators and a modern transformer that weighs less than traditional transformers. Also, the portable generator 920 may include a shortened power cable that is about 3 feet long to carry the RF energy from the portable generator to the treatment device. This is an improvement over current RF generators that often have long power cables (e.g., 10 feet long or greater) because approximately 10% of energy is lost for every foot of cable used, resulting in great energy inefficiency and making it necessary for the generator to be more powerful (and consume more energy) to compensate for the loss of energy transmitted.

The portable generator 920 may have a weight of about 10 pounds or less, or about 5 pounds or less. Also, in some embodiments, the portable generator 920 may have dimensions of about 5 inches in height, 10 inches in length, and 8 inches in depth, or less, or dimensions of about 3 inches in height, 8 inches in length, and 6 inches in depth, or less.

In one embodiment, the portable generator 920 has a line frequency of 50-60Hz, an output frequency of 348-460 kHz, a line voltage of 100-240 VAC, an output power of bipolar energy of 40-60 watts, and a passive cooling system. In this example, the portable generator 920 may provide treatment to a target tissue at an output of about 50 watts, a peak max voltage of 950-2900 volts, a crest factor of 5.5+/- 20%, and a frequency of 343-460 kHz +/- 10%.

Accordingly, the portable generator 920 and the portable wound treatment system, as described above, enables patients to receive plasma-mediated coagulation or ablation treatment, depending on the voltage applied by the portable generator, in many settings, such as home visits by a nurse or even by patients themselves. Thus, the portable wound treatment system improves patient access to treatments that promote control of the bacterial load, re-epithelialization, neovascularization, and ultimately, earlier wound healing and closure.

## Claims

1. A portable wound treatment system (100) comprising:
a disposable treatment device (105, 305, 405, 505, 605, 705, 801) comprising:
a handle (110, 310, 410, 510);
an interior cavity (112, 312, 412, 512, 612) for receiving a conductive fluid;
a plate (114, 314, 414, 514, 614, 814) configured to contact target tissue and coupled to a distal end of the handle, the plate enclosing the interior cavity to thereby retain the conductive fluid within the interior cavity and comprising an exterior surface (115, 315, 415, 515, 615, 815) having protrusions (619) that facilitate debridement of the target tissue as the exterior surface (115, 315, 415, 515, 615, 815) of the plate (114, 314, 414, 514, 614, 814) contacts the target tissue;
a conductive element (318, 518) disposed entirely within the interior cavity (112, 312, 412, 512, 612); and
a plurality of apertures (116, 316, 416, 516, 816) in the exterior surface of the plate (114, 314, 414, 514, 614, 814) configured to allow passage of the conductive fluid; and
a portable generator (920) configured to generate and transmit energy to the conductive element (318, 518) such that the conductive element (318, 518) conducts energy to be carried by the conductive fluid passing through one or more of the plurality of apertures (116, 316, 416, 516, 816) to treat a target tissue contacted by that conductive fluid.

2. The portable wound treatment system (100) of claim 1, wherein the plurality of apertures (116, 316, 416, 516, 816) are sized such that the conductive fluid does not flow from the plurality of apertures (116, 316, 416, 516, 816) due to gravity alone.

3. The portable wound treatment system (100) of claim 1, further comprising a non-conductive pad coupled (808) to the exterior surface (115, 315, 415, 515, 615, 815) of the plate (114, 314, 414, 514, 614, 814), the non-conductive pad (808) comprising an opening shaped or sized to correspond to a surface area of the target tissue or a dressing applied to the target tissue, and optionally
wherein the opening of the non-conductive pad (808) is shaped or sized such that the treatment device (105, 305, 405, 505, 605, 705, 801) selectively treats only a portion of the target tissue.

4. The portable wound treatment system (100) of any preceding claim, further comprising a reservoir (130) operable to contain the conductive fluid and to convey the conductive fluid to the conductive element (318, 518), and optionally further comprising an actuator (135) operable to control pressurized flow of the conductive fluid to the conductive element (318, 518) from the reservoir (130).

5. The portable wound treatment system (100) of any preceding claim, wherein the portable generator (920) generates radiofrequency, RF, energy.

6. The portable wound treatment system (100) of any preceding claim, wherein the conductive element (318, 518) is a bipolar helical coil.

7. The portable wound treatment system (100) of any preceding claim, further comprising a foam dressing (790B) that is applied to cover the target tissue prior to treatment with the wound treatment system (100), and to thereby improve conductivity to the target tissue, through the foam dressing (790B).

8. The portable wound treatment system (100) of any preceding claim, further comprising a suction device in fluid connection with the interior cavity (112, 312, 412, 512, 612) via tubing (650) coupled to the interior cavity (112, 312, 412, 512, 612).

9. The portable wound treatment system (100) of claim 8, further comprising a controller operable to adjust negative pressure produced by the suction device, and/or
wherein the suction device is operable to remove debris from the target tissue, as the target tissue is treated by the wound treatment system (100), by applying negative pressure to the treatment device (105, 305, 405, 505, 605, 705, 801).

10. The portable wound treatment system (100) of any preceding claim, wherein the portable generator (920) has a weight of about 2.3 kg or less, and/or
wherein the portable generator (920) has dimensions of about 12.7 cm in height, 25.4 cm in length, and 20.3 cm in depth, or less.

11. The portable wound treatment system (100) of claim 1, wherein the portable generator (920) is coupled to the conductive element (318, 518) by a power cable having a length of about 1.5 m or less, and optionally
wherein the portable generator (920) is coupled to the conductive element (318, 518) by a power cable having a length of about 0.9 m or less.

## Patentansprüche

1. Tragbares Wundbehandlungssystem (100), umfassend:
eine Einwegbehandlungsvorrichtung (105, 305, 405, 505, 605, 705, 801), umfassend:
einen Griff (110, 310, 410, 510);
einen inneren Hohlraum (112, 312, 412, 512, 612) zum Aufnehmen eines leitenden Fluids;
eine Platte (114, 314, 414, 514, 614, 814), die dazu konfiguriert ist, Zielgewebe zu kontaktieren, und an ein distales Ende des Griffes gekoppelt ist, wobei die Platte den inneren Hohlraum umschließt, um dadurch das leitende Fluid innerhalb des inneren Hohlraums zu halten, und umfassend eine äußere Oberfläche (115, 315, 415, 515, 615, 815), die Vorsprünge (619) aufweist, die ein Debridement des Zielgewebes ermöglichen, wenn die äußere Oberfläche (115, 315, 415, 515, 615, 815) der Platte (114, 314, 414, 514, 614, 814) das Zielgewebe kontaktiert;
ein leitendes Element (318, 518), das sämtlich innerhalb des inneren Hohlraums (112, 312, 412, 512, 612) angeordnet ist; und
eine Vielzahl von Öffnungen (116, 316, 416, 516, 816) in der äußeren Oberfläche der Platte (114, 314, 414, 514, 614, 814), die dazu konfiguriert ist, einen Durchgang des leitenden Fluids zu erlauben; und
einen tragbaren Erzeuger (920), der dazu konfiguriert ist, Energie zu erzeugen und derart an das leitende Element (318, 518) zu übertragen, dass das leitende Element (318, 518) Energie leitet, die durch das leitende Fluid zu befördern ist, das durch eine oder mehrere der Vielzahl von Öffnungen (116, 316, 416, 516, 816) durchgeht, um ein Zielgewebe zu behandeln, das durch jenes leitende Fluid kontaktiert wird.

2. Tragbares Wundbehandlungssystem (100) nach Anspruch 1, wobei die Vielzahl von Öffnungen (116, 316, 416, 516, 816) derart bemessen ist, dass das leitende Fluid nicht lediglich aufgrund von Schwerkraft aus der Vielzahl von Öffnungen (116, 316, 416, 516, 816) fließt.

3. Tragbares Wundbehandlungssystem (100) nach Anspruch 1, ferner umfassend ein nicht leitendes Kissen (808), das an die äußere Oberfläche (115, 315, 415, 515, 615, 815) der Platte (114, 314, 414, 514, 614, 814) gekoppelt ist, wobei das nicht leitende Kissen (808) ein Loch umfasst, das dazu geformt oder bemessen ist, einem Oberflächenbereich des Zielgewebes oder einem Verband, der auf das Zielgewebe angelegt ist, zu entsprechen, und gegebenenfalls
wobei das Loch des nicht leitenden Kissens (808) derart geformt oder bemessen ist, dass die Behandlungsvorrichtung (105, 305, 405, 505, 605, 705, 801) selektiv nur einen Abschnitt des Zielgewebes behandelt.

4. Tragbares Wundbehandlungssystem (100) nach einem der vorhergehenden Ansprüche, ferner umfassend ein Reservoir (130), das dazu betreibbar ist, das leitende Fluid zu halten und das leitende Fluid zu dem leitenden Element (318, 518) zu führen, und gegebenenfalls ferner umfassend einen Aktor (135), der dazu betreibbar ist, einen druckbeaufschlagten Fluss des leitenden Fluids zu dem leitenden Element (318, 518) aus dem Reservoir (130) zu steuern.

5. Tragbares Wundbehandlungssystem (100) nach einem der vorhergehenden Ansprüche, wobei der tragbare Erzeuger (920) Hochfrequenzenergie, RF-Energie, erzeugt.

6. Tragbares Wundbehandlungssystem (100) nach einem der vorhergehenden Ansprüche, wobei das leitende Element (318, 518) eine bipolare Spiralfeder ist.

7. Tragbares Wundbehandlungssystem (100) nach einem der vorhergehenden Ansprüche, ferner umfassend einen Schaumverband (790B), der angelegt wird, um das Zielgewebe vor der Behandlung mit dem Wundbehandlungssystem (100) zu bedecken und dadurch die Leitfähigkeit zu dem Zielgewebe durch den Schaumverband (790B) hindurch zu verbessern.

8. Tragbares Wundbehandlungssystem (100) nach einem der vorhergehenden Ansprüche, ferner umfassend eine Saugvorrichtung in Fluidverbindung mit dem inneren Hohlraum (112, 312, 412, 512, 612), die über einen Schlauch (650) an den inneren Hohlraum (112, 312, 412, 512, 612) gekoppelt ist.

9. Tragbares Wundbehandlungssystem (100) nach Anspruch 8, ferner umfassend eine Steuerung, die dazu betreibbar ist, einen negativen Druck, der durch die Saugvorrichtung produziert wird, einzustellen, und/oder
wobei die Saugvorrichtung dazu betreibbar ist, Ablagerungen, wenn das Zielgewebe durch das Wundbehandlungssystem (100) behandelt wird, durch Anlegen des negativen Druckes auf die Behandlungsvorrichtung (105, 305, 405, 505, 605, 705, 801) aus dem Zielgewebe zu entfernen.

10. Tragbares Wundbehandlungssystem (100) nach einem der vorhergehenden Ansprüche, wobei der tragbare Erzeuger (920) ein Gewicht von ungefähr 2,3 kg oder weniger aufweist und/oder
wobei der tragbare Erzeuger (920) Abmessungen von ungefähr 12,7 cm Höhe, 25,4 cm Länge und 20,3 cm Tiefe oder weniger aufweist.

11. Tragbares Wundbehandlungssystem (100) nach Anspruch 1, wobei der tragbare Erzeuger (920) durch ein Stromkabel, das eine Länge von ungefähr 1,5 m oder weniger aufweist, an das leitende Element (318, 518) gekoppelt ist und gegebenenfalls
wobei der tragbare Erzeuger (920) durch ein Stromkabel, das eine Länge von ungefähr 0,9 m oder weniger aufweist, an das leitende Element (318, 518) gekoppelt ist.

## Revendications

1. Système portable de traitement de plaies (100) comprenant :
un dispositif de traitement jetable (105, 305, 405, 505, 605, 705, 801) comprenant :
une poignée (110, 310, 410, 510) ;
une cavité intérieure (112, 312, 412, 512, 612) destinée à recevoir un fluide conducteur ;
une plaque (114, 314, 414, 514, 614, 814) configurée pour entrer en contact avec le tissu cible et couplée à une extrémité distale de la poignée, la plaque entourant la cavité intérieure pour ainsi retenir le fluide conducteur à l'intérieur de la cavité intérieure et comprenant une surface extérieure (115, 315, 415, 515, 615, 815) ayant des saillies (619) qui facilitent le débridement du tissu cible lorsque la surface extérieure (115, 315, 415, 515, 615, 815) de la plaque (114, 314, 414, 514, 614, 814) entre en contact avec le tissu cible ;
un élément conducteur (318, 518) disposé entièrement à l'intérieur de la cavité intérieure (112, 312, 412, 512, 612) ; et
une pluralité d'ouvertures (116, 316, 416, 516, 816) dans la surface extérieure de la plaque (114, 314, 414, 514, 614, 814) configurées pour permettre le passage du fluide conducteur ; et
un générateur portable (920) configuré pour générer et transmettre de l'énergie à l'élément conducteur (318, 518) de sorte que l'élément conducteur (318, 518) conduise l'énergie devant être transportée par le fluide conducteur passant à travers une ou plusieurs de la pluralité d'ouvertures (116, 316, 416, 516, 816) pour traiter un tissu cible en contact avec ce fluide conducteur.

2. Système de traitement de plaies portable (100) selon la revendication 1, dans lequel la pluralité d'ouvertures (116, 316, 416, 516, 816) sont dimensionnées de sorte que le fluide conducteur ne s'écoule pas de la pluralité d'ouvertures (116, 316, 416, 516, 816) en raison de la gravité seule.

3. Système de traitement de plaies portable (100) selon la revendication 1, comprenant en outre un tampon non conducteur couplé (808) à la surface extérieure (115, 315, 415, 515, 615, 815) de la plaque (114, 314, 414, 514, 614, 814), le tampon non conducteur (808) comprenant une ouverture façonnée ou dimensionnée pour correspondre à une zone de surface du tissu cible ou d'un pansement appliqué sur le tissu cible, et éventuellement
dans lequel l'ouverture du tampon non conducteur (808) est façonnée ou dimensionnée de sorte que le dispositif de traitement (105, 305, 405, 505, 605, 705, 801) traite sélectivement seulement une partie du tissu cible.

4. Système de traitement de plaies portable (100) selon l'une quelconque des revendications précédentes, comprenant en outre un réservoir (130) pouvant fonctionner pour contenir le fluide conducteur et à transporter le fluide conducteur vers l'élément conducteur (318, 518), et comprenant en outre éventuellement un actionneur (135) pouvant fonctionner pour contrôler l'écoulement sous pression du fluide conducteur vers l'élément conducteur (318, 518) à partir du réservoir (130).

5. Système de traitement de plaies portable (100) selon l'une quelconque des revendications précédentes, dans lequel le générateur portable (920) génère de l'énergie radiofréquence, RF.

6. Système de traitement de plaies portable (100) selon l'une quelconque des revendications précédentes, dans lequel l'élément conducteur (318, 518) est une bobine hélicoïdale bipolaire.

7. Système de traitement de plaies portable (100) selon l'une quelconque des revendications précédentes, comprenant en outre un pansement en mousse (790B) qui est appliqué pour recouvrir le tissu cible avant le traitement avec le système de traitement de plaies (100), et pour ainsi améliorer la conductivité vers le tissu cible, à travers le pansement en mousse (790B).

8. Système de traitement de plaies portable (100) selon l'une quelconque des revendications précédentes, comprenant en outre un dispositif d'aspiration en connexion fluidique avec la cavité intérieure (112, 312, 412, 512, 612) via un tube (650) couplé à la cavité intérieure (112, 312, 412, 512, 612).

9. Système de traitement de plaies portable (100) selon la revendication 8, comprenant en outre un contrôleur pouvant fonctionner pour ajuster la pression négative produite par le dispositif d'aspiration, et/ou
dans lequel le dispositif d'aspiration peut fonctionner pour éliminer les débris du tissu cible, lorsque le tissu cible est traité par le système de traitement de plaies (100), en appliquant une pression négative au dispositif de traitement (105, 305, 405, 505, 605, 705, 801).

10. Système de traitement de plaies portable (100) selon l'une quelconque des revendications précédentes, dans lequel le générateur portable (920) a un poids d'environ 2,3 kg ou moins, et/ou dans lequel le générateur portable (920) a des dimensions d'environ 12,7 cm de hauteur, 25,4 cm de longueur et 20,3 cm de profondeur, ou moins.

11. Système de traitement de plaies portable (100) selon la revendication 1, dans lequel le générateur portable (920) est couplé à l'élément conducteur (318, 518) par un câble d'alimentation ayant une longueur d'environ 1,5 m ou moins, et éventuellement dans lequel le générateur portable (920) est couplé à l'élément conducteur (318, 518) par un câble d'alimentation ayant une longueur d'environ 0,9 m ou moins.
